# EUROPEAN PATENT SPECIFICATION

(11) **EP 4 051 663 B1**
(45) Date of publication and mention of the grant of the patent: **21.02.2024**
(21) Application number: 20800097.6
(22) Date of filing: 29.10.2020
(51) Int. Cl.: C07D 213/74

(54) **SYNTHESIS OF 3-NITRO-N-(2,2,2-TRIFLUOROETHYL)-4-PYRIDINAMINE**
SYNTHESE VON 3-NITRO-N(2,2,2-TRIFLUORETHYL)-4-PYRIDINAMIN
SYNTHÈSE DE 3-NITRO-N-(2,2,2-TRIFLUOROÉTHYL)-4-PYRIDINAMINE

(30) Priority: 30.10.2019 WO PCT/CN2019/114253
(43) Date of publication of application: 07.09.2022
(73) Proprietor: Janssen Sciences Ireland Unlimited Company, Ringaskiddy, Co Cork (IE)
(72) Inventor: TAN, Hongyu, Shanghai (CN); HAN, Licheng, Shanghai (CN)
(74) Representative: Verberckmoes, Filip Gerard
(86) International application number: PCT/EP2020/080379
(87) International publication number: WO 2021/083996

(56) References cited:
- WO-A1-01/95910
- WO-A1-2014/081718
- WO-A1-2018/187480

## Description

The present invention relates to a chemical synthesis route for preparing 3-nitro-*N*-(2,2,2-trifluoroethyl)-4-pyridinamine which compound can be used as an intermediate compound in the synthesis of RSV inhibiting imidazopyridines or imidazopyrimidines.

### Background

Respiratory syncytial virus is a major cause of acute lower respiratory tract infection in young children, immunocompromised adults, and the elderly. Intervention with small-molecule antivirals specific for respiratory syncytial virus presents an important therapeutic opportunity, but no such compounds are approved today.

3-Nitro-*N*-(2,2,2-trifluoroethyl)-4-pyridinamine is a compound that can be used as a building block in the synthesis of RSV inhibiting imidazopyridines or imidazopyrimidines such as those disclosed in WO-01/95910.

### Prior art

WO-01/95910 discloses on page 65 the hydrochloric acid salt of 3-nitro-*N*-(2,2,2-trifluoroethyl)-4-pyridinamine which is prepared from 4-methoxy-3-nitro-pyridine as depicted below:

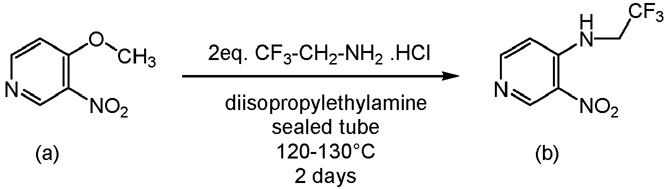

### Description of the invention

It has now been found that 3-nitro-*N*-(2,2,2-trifluoroethyl)-4-pyridinamine can be prepared conveniently by treating 4-methoxy-3-nitro-pyridine with 2,2,2-trifluoroethylamine in the presence of an aqueous solution comprising an acid under heating.

For large scale production this new synthesis method avoids the use of a high-pressure reaction vessel (*i.e*. the upscaled version of a sealed tube) and reduced reaction time from 2 days to less than one day (typically between 10 to 16 hours).

The present invention provides a process for preparing 3-nitro-*N*-(2,2,2-trifluoroethyl)-4-pyridinamine of formula (b) by treating 4-methoxy-3-nitro-pyridine of formula (a) with 2,2,2-trifluoroethylamine in the presence of an aqueous solution comprising an acid under heating.

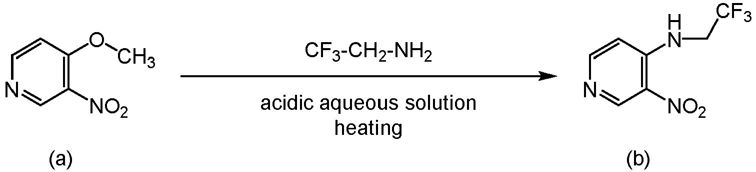

The acid used can be any inorganic or organic acid that is soluble in water and is capable of protonating the pyridine moiety to activate the pyridine ring for nucleophilic substitution. Inorganic acids are selected from hydrochloric acid, hydrobromic acid, sulfuric acid, phosphoric acid, boric acid, nitric acid, and the like. Organic acids are preferably carboxylic acids selected from formic acid, acetic acid, p-toluenesulfonic acid, citric acid, oxalic acid, malonic acid, succinic acid, lactic acid, and the like. A suitable organic acid is citric acid, or citric acid monohydrate, which has a low cost, low toxicity and easy to handle. The amount of organic acid can be any amount between 1 mole to 5 mole with respect to the amount of 4-methoxy-3-nitro-pyridine. In practice the amount of organic acid ranges from 1.2 to 3 mole and a preferred range is 1.2 to 1.5.

The amount of 2,2,2-trifluoroethylamine can be any amount between 1 mole to 5 mole with respect to the amount of 4-methoxy-3-nitro-pyridine. In practice the amount of 2,2,2-trifluoroethylamine ranges from 1.1 to 3 mole.

The reaction may conveniently be carried out at a temperature ranging between room temperature and the reflux temperature of the reaction mixture. In practice the temperature can range from room temperature to 90°C, or from 30°C to 80°C, or from 40°C to 70°C.

Upon completion of the reaction, the desired 3-nitro-*N*-(2,2,2-trifluoroethyl)-4-pyridinamine can be isolated from the reaction mixture by extraction with an organic solvent.

### Example : synthesis of 3-nitro-N-(2,2,2-trifluoroethyl)-4 pyridinamine

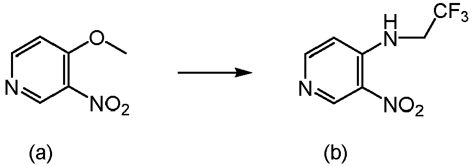

Citric acid monohoydrate (295.0 g) is dissolved in water (370.0 g) and 4-methoxy-3-nitropyridine (179.0 g) is then added followed by 2,2,2-trifluoroethylamine (348.0 g). The mixture is stirred at 50°C until complete conversion (10-16 hours). After cooling to room temperature, 2-MeTHF (1250 mL) is added and the mixture is stirred for 1 hour and the phases are then separated. The aqueous phase is re-extracted with 2-MeTHF (530 mL). The combined organic layers are washed with 7% aqueous NaHCO₃ solution (890 g) and finally with water (903 g). The organic layer is evaporated to dryness yielding 3-nitro-*N*-(2,2,2-trifluoroethyl)-4-pyridinamine (233.7 g, 91% yield).
¹H NMR (400 MHz, DMSO-d₆) δ ppm 4.28 - 4.47 (m, 2 H); 7.28 (d, *J* = 6.27 Hz, 1 H); 8.38 (d, *J* = 6.02 Hz, 1 H); 8.63 (br t, J=6.65 Hz, 1 H); 9.08 (s, 1 H)
¹³C NMR (101 MHz, DMSO-d₆) δ ppm 43.15 (q, J=33.0 Hz); 109.30 124.05; 126.84; 130.70; 148.55 (d, J=4.4 Hz); 153.68

## Claims

1. A process for preparing 3-nitro-*N*-(2,2,2-trifluoroethyl)-4-pyridinamine of formula (b) 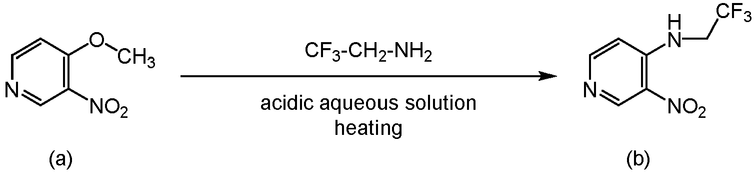 by treating 4-methoxy-3-nitro-pyridine of formula (a) with 2,2,2-trifluoroethylamine in an aqueous solution comprising an acid under heating.

2. The process as claimed in claim 1 wherein the acid is an inorganic acid or an organic acid.

3. The process as claimed in claim 2 wherein the inorganic acid is selected from the list consisting of hydrochloric acid, hydrobromic acid, sulfuric acid, phosphoric acid, boric acid, and nitric acid.

4. The process as claimed in claim 2 wherein the organic acid selected from the list consisting of formic acid, acetic acid, p-toluenesulfonic acid, citric acid, oxalic acid, malonic acid, succinic acid, and lactic acid.

5. The process as claimed in claim 4 wherein the organic acid is citric acid.

6. The process as claimed in any one of claims 1 to 5 wherein the reaction mixture is heated to a temperature ranging from room temperature to the reflux temperature of the reaction mixture.

7. The process as claimed in claim 6 wherein the temperature ranges from 30°C to 80°C.

8. The process as claimed in claim 7 wherein the temperature ranges from 40°C to 70°C.

9. The process as claimed in any one of the preceding claims wherein the amount of organic acid ranges from 1 mole to 5 mole and the amount of 2,2,2-trifluoroethylamine ranges from 1 to 3 mole.

## Patentansprüche

1. Verfahren zum Herstellen von 3-Nitro-*N*-(2,2,2-trifluorethyl)-4-pyridinamin von Formel (b) 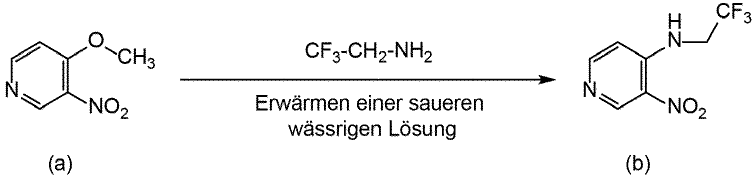 durch Behandeln von 4-Methoxy-3-nitro-pyridin von Formel (a) mit 2,2,2-Trifluorethylamin in einer wässrigen Lösung, umfassend eine Säure, unter Erwärmen.

2. Verfahren nach Anspruch 1, wobei die Säure eine anorganische Säure oder eine organische Säure ist.

3. Verfahren nach Anspruch 2, wobei die anorganische Säure aus der Liste ausgewählt ist, bestehend aus Chlorwasserstoffsäure, Bromwasserstoffsäure, Schwefelsäure, Phosphorsäure, Borsäure und Salpetersäure.

4. Verfahren nach Anspruch 2, wobei die organische Säure aus der Liste ausgewählt ist, bestehend aus Ameisensäure, Essigsäure, p-Toluolsulfonsäure, Citronensäure, Oxalsäure, Malonsäure, Bernsteinsäure und Milchsäure.

5. Verfahren nach Anspruch 4, wobei die organische Säure Citronensäure ist.

6. Verfahren nach einem der Ansprüche 1 bis 5, wobei das Reaktionsgemisch auf eine Temperatur erwärmt wird, die von Raumtemperatur bis zu der Rückflusstemperatur des Reaktionsgemisches reicht.

7. Verfahren nach Anspruch 6, wobei die Temperatur von 30 °C bis 80 °C reicht.

8. Verfahren nach Anspruch 7, wobei die Temperatur von 40 °C bis 70 °C reicht.

9. Verfahren nach einem der vorstehenden Ansprüche, wobei die Menge an organischer Säure von 1 Mol bis 5 Mol reicht, und die Menge an 2,2,2-Trifluorethylamin von 1 bis 3 Mol reicht.

## Revendications

1. Procédé de préparation de 3-nitro-*N*-(2,2,2-trifluoroéthyl)-4-pyridinamine de formule (b) 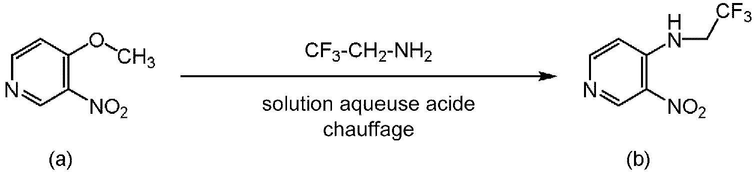 en traitant 4-méthoxy-3-nitro-pyridine de formule (a) avec 2,2,2-trifluoroéthylamine dans une solution aqueuse comprenant un acide sous chauffage.

2. Procédé selon la revendication 1, dans lequel l'acide est un acide inorganique ou un acide organique.

3. Procédé selon la revendication 2, dans lequel l'acide inorganique est choisi dans la liste constituée par acide chlorhydrique, acide bromhydrique, acide sulfurique, acide phosphorique, acide borique et acide nitrique.

4. Procédé selon la revendication 2, dans lequel l'acide organique est choisi dans la liste constituée par acide formique, acide acétique, acide p-toluènesulfonique, acide citrique, acide oxalique, acide malonique, acide succinique et acide lactique.

5. Procédé selon la revendication 4, dans lequel l'acide organique est acide citrique.

6. Procédé selon l'une quelconque des revendications 1 à 5, dans lequel le mélange réactionnel est chauffé à une température allant de la température ambiante à la température de reflux du mélange réactionnel.

7. Procédé selon la revendication 6, dans lequel la température va de 30 °C à 80 °C.

8. Procédé selon la revendication 7, dans lequel la température va de 40 °C à 70 °C.

9. Procédé selon l'une quelconque des revendications précédentes, dans lequel la quantité d'acide organique va de 1 mole à 5 moles et la quantité de 2,2,2-trifluoroéthylamine va de 1 à 3 moles.
